# EUROPEAN PATENT APPLICATION

(11) **EP 4 338 726 A1**
(43) Date of publication of application: **20.03.2024**
(21) Application number: 22807302.9
(22) Date of filing: 14.04.2022
(51) Int. Cl.: A61K 8/898, A61K 8/31, A61K 8/37, A61K 8/60, A61K 8/81, A61Q 1/04

(54) **OILY COSMETIC**

(30) Priority: 13.05.2021 JP 2021081342
(71) Applicant: SHIN-ETSU CHEMICAL CO., LTD., Tokyo 1000005 (JP)
(72) Inventor: MORIYA Hiroyuki, Annaka-shi, Gunma 379-0224 (JP); TANAKA Mituru, Tokyo 100-0005 (JP)
(74) Representative: Angerhausen, Christoph
(86) International application number: PCT/JP2022/017813
(87) International publication number: WO 2022/239599

(57) **Abstract**

Developed is an oily cosmetic imparting an adhesiveness, softness, smoothness, luster, and emollient effect, bringing about a natural makeup finish, and ensuring an excellent makeup durability without showing color transfer and color fade, while having a light spreadability, non-sticky feeling of use, and superior water repellency. The oily cosmetic contains components (A) and (B) which are: (A) an organopolysiloxane compound that has a weight-average molecular weight of 500 to 200,000; and (B) a hydrocarbon-based oily solidifying agent and/or a non-hydrocarbon-based oily gelling agent.

## Description

### TECHNICAL FIELD

The present invention relates to an oily cosmetic containing a particular siloxane compound.

### BACKGROUND ART

Conventionally, as oily cosmetics, there have been used those containing oil agents such as paraffin, esters, higher alcohols, and glycerides for the purposes of imparting a softness and smoothness, and bringing about an emollient effect thereby. However, these cosmetics inevitably have an oily, sticky, or oil membrane-like feeling. There, in order to suppress such stickiness, there are cosmetics also employing oil agents having a high molecular weight and a low unsaturation degree; however, the problems with these cosmetics are that peeling will occur due to an insufficient adhesiveness, and hardness will occur as well.

Further, there are also known cosmetics prepared by adding silicone oils such as dimethylpolysiloxane to the abovementioned oily cosmetics. However, while silicone oils are capable of imparting a light spreadability and a more excellent smoothness and water repellency, they have a poor compatibility with other oil agents, which has led to an inferior adhesiveness and thereby caused silicone oil-containing cosmetics to have a downside of being inferior in makeup durability.

Meanwhile, in the cases of lip cosmetics amongst oily cosmetics, various studies have been conducted heretofore to improve adhesiveness. For example, there are attempts to add a volatile oil agent so that makeup durability can be improved by allowing the liquid oil agents to be volatilized after application, and only the color materials and wax ingredients to remain on the coated surface. Further, there are also attempts to improve makeup durability by dissolving and adding a polymer membrane-forming agent into a volatile oil agent, and then forming a polymer membrane on the skin surface after application and drying. In addition, studies are also being made to improve makeup durability by, for example, adding a PCA-modified dimethicone containing pyrrolidone carboxyl groups (pyrrolidone carboxylic acid residues) that bring about a favorable adhesiveness (Patent documents 1 and 2).

However, in the case of a volatile oil agent-containing oily cosmetic, a feeling of dryness and a declining luster will be observed on the skin, or a lack of adhesiveness will occur. Further, if adding a PCA-modified dimethicone, a poor compatibility will be observed with respect to a hydrocarbon-based oily solidifying agent and non-hydrocarbon-based oily gelling agent serving as the skeleton of an oily cosmetic. In order to add a volatile oil agent or PCA-modified dimethicone to an oily cosmetic, it is necessary that there is also added a polyoxyalkylene-modified organopolysiloxane or the like. However, a polyoxyalkylene-modified organopolysiloxane disperses the solidifying agent and gelling agent used in an oily cosmetic, and therefore makes it impossible to maintain the hardness of the oily cosmetic.

### PRIOR ART DOCUMENTS

### Patent documents

Patent document 1: JP-A-2003-238334
Patent document 2: JP-A-2003-261415

### SUMMARY OF THE INVENTION

### Problems to be solved by the invention

Thus, there has been a demand to develop an oily cosmetic imparting an adhesiveness, softness, smoothness, luster, and emollient effect, bringing about a natural makeup finish, and ensuring an excellent makeup durability without showing color transfer and color fade, while having a light spreadability, non-sticky feeling of use, and superior water repellency. Particularly, in the field of lip cosmetics, there has been a demand to develop an oily cosmetic realizing a moist makeup finish without causing dryness on the lip, and bringing about an excellent luster and adhesiveness as well as a favorable makeup durability without exhibiting smudge.

### Means to solve the problems

As a result of diligently conducting a series of studies to solve the above problems, the inventors of the present invention found that an oily cosmetic containing a pyrrolidone carboxyl group and aromatic group-containing organopolysiloxane compound, and an oily solidifying agent and/or oily gelling agent, was superior in formability, and had a light spreadability, a non-sticky feeling of use as well as a strong water repellency to sweat and water. Further, it became clear to the inventors that this oily cosmetic was able to impart a softness, smoothness, emollient effect, luster, and natural makeup finish, and had a feature of being superior in makeup durability without showing color transfer and color fade. Particularly, the inventors made the present invention by finding that a lip cosmetic containing this oily cosmetic was able to impart an emollient effect to the lip and bring about a favorable make durability with an excellent luster an adhesiveness.

That is, the present invention is to provide the following oily cosmetic.

[1] An oily cosmetic comprising the following components (A) and (B):
   (A) an organopolysiloxane compound that is represented by the following general formula (1) and has a weight-average molecular weight of 500 to 200,000,
      [Chemical formula 1]

      R¹ₐR²_{b}R³_{c}R⁴_{d}SiO_{(4-a-b-c-d)/2}··· (1)

      wherein R¹ independently represents an alkyl group having 1 to 16 carbon atoms, or a fluorine-substituted alkyl group having 3 to 12 carbon atoms; R² independently represents an organic group expressed by the following general formula (2); R³ independently represents an aryl group having 6 to 12 carbon atoms, or an aralkyl group having 7 to 14 carbon atoms; R⁴ independently represents an organopolysiloxane group expressed by the following general formula (3) or (4); a, b, c and d satisfy 1.0≤a≤2.5, 0.001≤b≤1.5, 0.001≤c≤1.5, 0≤d≤1.0, and 1.1<a+b+c+d<4, 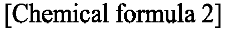 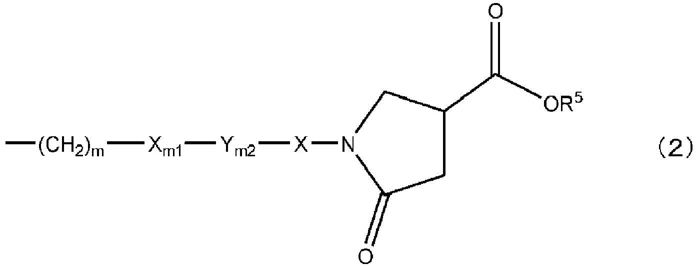 wherein R⁵ represents a hydrogen atom, a lower alkyl group having 1 to 6 carbon atoms, or an alkali metal cation; X independently represents a linear or branched alkylene group having 1 to 12 carbon atoms; Y represents -NR'-, a sulfur atom, or an oxygen atom, in which R' represents a hydrogen atom or a lower alkyl group having 1 to 6 carbon atoms; m is 0 or 2, m1 is 0 or 1, and m2 is 0 or 1, provided that when m is 0 and m2 is 1, m1 is 1, that when m is 2 and m2 is 1, m1 is 0 or 1, and that when m is 2 and m2 is 0, m1 is 0,
      [Chemical formula 3]

      MM^{R}Dₛ (3)

      M_{q}DₛD^{R}ₜT^{R}ᵤ (4)

      wherein M=R⁶₃SiO_{1/2}, M^{R}=R⁶₂R⁷SiO_{1/2}, D=R⁶₂SiO_{2/2}, D^{R}=R⁶R⁷SiO_{2/2}, T^{R}=R⁷SiO_{3/2}; R⁶ independently represents a group selected from an alkyl group having 1 to 6 carbon atoms, an aryl group having 6 to 12 carbon atoms, an aralkyl group having 7 to 14 carbon atoms, and a fluorine-substituted alkyl group having 3 to 12 carbon atoms; R⁷ independently represents an alkylene group having 1 to 5 carbon atoms; q is an integer of 2 to 3; s satisfies 0≤s≤500; t and u are each a number of 0 or 1, provided that in the formula (4), a sum of t and u is 1, and s and u shall not both be 1 or larger at the same time;
   (B) a hydrocarbon-based oily solidifying agent and/or a non-hydrocarbon-based oily gelling agent.
[2] The oily cosmetic according to [1], wherein the hydrocarbon-based oily solidifying agent as the component (B) is at least one kind of oily solidifying agent selected from a paraffin wax, a polyethylene wax, a polyethylene/polypropylene wax, a Fischer-Tropsch wax, ceresin, and a microcrystalline wax.
[3] The oily cosmetic according to [1], wherein the non-hydrocarbon-based oily gelling agent as the component (B) is at least one kind of oily gelling agent selected from a sugar skeleton-containing fatty acid ester and a glycerin skeleton-containing fatty acid ester.
[4] The oily cosmetic according to any one of [1] to [3], wherein the oily cosmetic is a lip cosmetic.

### Effects of the invention

The oily cosmetic of the present invention is superior in formability, and is superior in usability in that no stickiness or heaviness is felt at the time of application, which contributes to a non-sticky and light spreadability; the skin treated has a non-sticky, smooth and moist feel; the cosmetic even has a strong water repellency to sweat and water; and as a result of applying the cosmetic, it is capable of imparting an adhesiveness, softness, smoothness, emollient effect, and luster to the skin, and bringing about a natural makeup finish. Particularly, when used in a lip cosmetic, the cosmetic of the present invention is able to impart a healthy luster, a moist makeup finish without causing dryness on the lip, and a makeup finish superior in durability without showing smudge, color transfer, and color fade due to a high adhesiveness.

### MODE FOR CARRYING OUT THE INVENTION

The present invention is described in detail hereunder.

### Component (A)

An organopolysiloxane compound that is used as a component (A) in the present invention and has a weight-average molecular weight of 500 to 200,000 is represented by the following general formula (1).
[Chemical formula 4]

R¹ₐR²_{b}R³cR⁴_{d}SiO_{(4-a-b-c-d)/2}··· (1)

[In this formula, R¹ independently represents an alkyl group having 1 to 16 carbon atoms, or a fluorine-substituted alkyl group having 3 to 12 carbon atoms; R² independently represents an organic group expressed by the following general formula (2); R³ independently represents an aryl group having 6 to 12 carbon atoms, or an aralkyl group having 7 to 14 carbon atoms; R⁴ independently represents an organopolysiloxane group expressed by the following general formula (3) or (4); a, b, c and d satisfy 1.0≤a≤2.5, 0.001≤b≤1.5, 0.001≤c≤1.5, 0≤d≤1.0, and 1.1<a+b+c+d<4.] 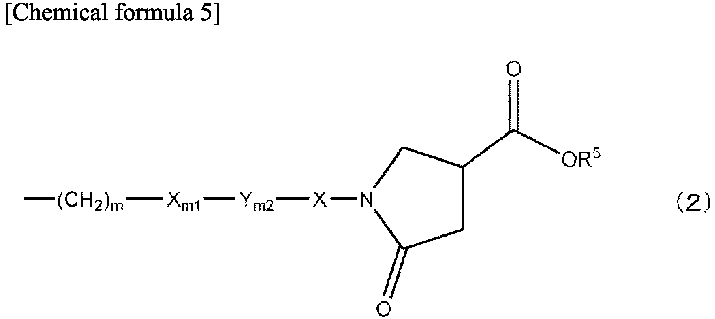 [In this formula, R⁵ represents any of a hydrogen atom, a lower alkyl group having 1 to 6 carbon atoms, or an alkali metal cation; X independently represents a linear or branched alkylene group having 1 to 12 carbon atoms; Y represents -NR'-, a sulfur atom, or an oxygen atom, in which R' represents a hydrogen atom or a lower alkyl group having 1 to 6 carbon atoms. m is 0 or 2, m1 is 0 or 1, and m2 is 0 or 1. However, when m is 0 and m2 is 1, m1 is 1; when m is 2 and m2 is 1, m1 is 0 or 1; when m is 2 and m2 is 0, m1 is 0.]
[Chemical formula 6]

MM^{R}Dₛ (3)

M_{q}DₛD^{R}ₜT^{R}ᵤ (4)

[In these formulae, M=R⁶₃SiO_{1/2}, M^{R}=R⁶₂R⁷SiO_{1/2}, D=R⁶₂SiO_{2/2}, D^{R}=R⁶R⁷SiO_{2/2}, T^{R}=R⁷SiO_{3/2}; R⁶ independently represents a group selected from an alkyl group having 1 to 6 carbon atoms, an aryl group having 6 to 12 carbon atoms, an aralkyl group having 7 to 14 carbon atoms, and a fluorine-substituted alkyl group having 3 to 12 carbon atoms; R⁷ independently represents an alkylene group having 1 to 5 carbon atoms. q is an integer of 2 to 3. s satisfies 0≤s≤500. t and u are each a number of 0 or 1. However, in the formula (4), a sum of t and u is 1, s and u shall not both be 1 or larger at the same time.]

Specific examples of the alkyl group represented by R¹ include a methyl group, an ethyl group, a propyl group, a butyl group, a pentyl group, a hexyl group, a heptyl group, an octyl group, a nonyl group, a decyl group, a dodecyl group, a hexadecyl group, a cyclopentyl group, and a cyclohexyl group. Specific examples of the fluorine-substituted alkyl group include a trifluoropropyl group, a nonafluorohexyl group, a tridecafluorooctyl group, and a heptadecafluorodecyl group. Further, it is preferred that 50% or more, more preferably 70% or more of all the R¹s are methyl groups. Further, it may also be that all the R¹s are methyl groups.

R² is an organic group represented by the following general formula (2).

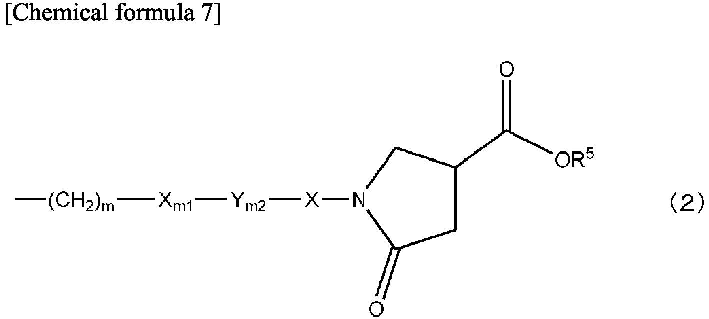

In this formula, R⁵ represents any of a hydrogen atom, a lower alkyl group having 1 to 6 carbon atoms, or an alkali metal cation. Examples of the lower alkyl group represented by R⁵ include a methyl group, an ethyl group, a propyl group, a butyl group, a pentyl group, and a hexyl group. Examples of the alkali metal cation include a sodium ion, a potassium ion, and a lithium ion. R⁵ is preferably a hydrogen atom, a methyl group, a sodium ion, or a potassium ion, more preferably a hydrogen atom. X independently represents a linear or branched alkylene group having 1 to 12 carbon atoms. Examples of the alkylene group represented by X include a methylene group, an ethylene group, and a trimethylene group. Y represents -NR'-, a sulfur atom, or an oxygen atom. Here, R' represents a hydrogen atom or a lower alkyl group having 1 to 6 carbon atoms. Examples of the lower alkyl group represented by R' include a methyl group, an ethyl group, a propyl group, a butyl group, a pentyl group, and a hexyl group. Among them, R' is preferably a hydrogen atom, a methyl group, or an ethyl group, more preferably a hydrogen atom. m is 0 or 2, m1 is 0 or 1, and m2 is 0 or 1. However, when m is 0 and m2 is 1, m1 is 1; when m is 2 and m2 is 1, m1 is 0 or 1; when m is 2 and m2 is 0, m1 is 0.

R³ independently represents an aryl group having 6 to 12 carbon atoms, or an aralkyl group having 7 to 14 carbon atoms. Examples of such aryl group include a phenyl group and a tolyl group; examples of the aralkyl group include a benzyl group and a phenethyl group. R³ is preferably a phenyl group.

R⁴ independently represents an organopolysiloxane group expressed by the following general formula (3) or (4).
[Chemical formula 8]

MM^{R}Dₛ (3)

M_{q}DₛD^{R}ₜT^{R}ᵤ (4)

[In these formulae, M=R⁶₃SiO_{1/2}, M^{R}=R⁶₂R⁷SiO_{1/2}, D=R⁶₂SiO_{2/2}, D^{R}=R⁶R⁷SiO_{2/2}, T^{R}=R⁷SiO_{3/2}; R⁶ independently represents a group selected from an alkyl group having 1 to 6 carbon atoms, an aryl group having 6 to 12 carbon atoms, an aralkyl group having 7 to 14 carbon atoms, and a fluorine-substituted alkyl group having 3 to 12 carbon atoms; R⁷ represents an alkylene group having 1 to 5 carbon atoms, q is an integer of 2 to 3. s satisfies 0≤s≤500. t and u are each a number of 0 or 1. However, in the formula (4), a sum of t and u is 1, and s and u shall not both be 1 or larger at the same time.]

Specific examples of the organopolysiloxane group represented by the general formula (3) or (4) are as follows. 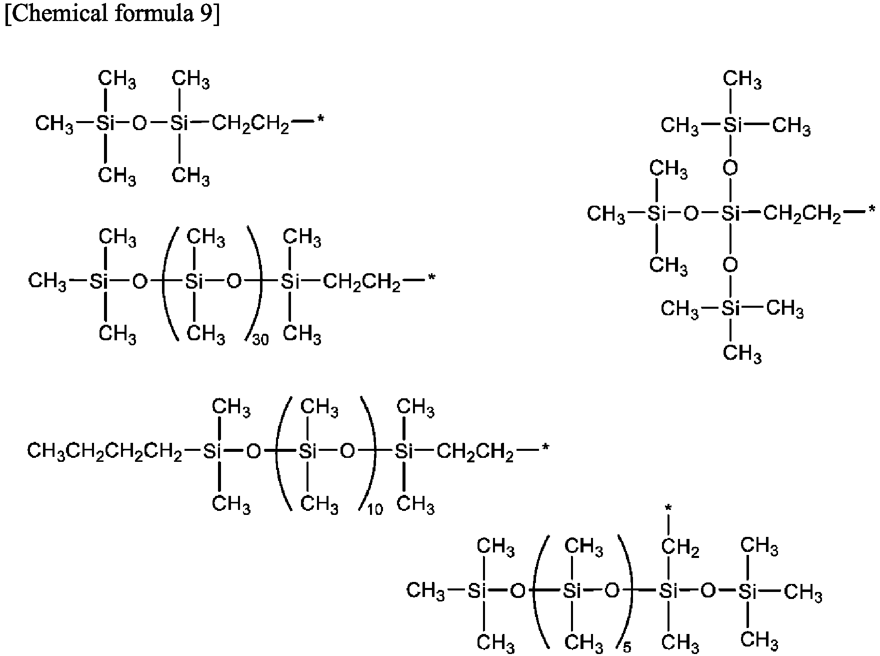 (In the above formulae, * are bonding sites where R⁴ binds to other silicon atom.)

R⁶ independently represents a group selected from an alkyl group having 1 to 6 carbon atoms, an aryl group having 6 to 12 carbon atoms, an aralkyl group having 7 to 14 carbon atoms, and a fluorine-substituted alkyl group having 3 to 12 carbon atoms. Specific examples thereof may include alkyl groups such as a methyl group, an ethyl group, a propyl group, a butyl group, a pentyl group, a hexyl group, and a cyclohexyl group; aryl groups such as a phenyl group and a tolyl group; aralkyl groups such as a benzyl group and a phenethyl group; and fluorine-substituted alkyl groups such as a trifluoropropyl group, a nonafluorohexyl group, a tridecafluorooctyl group, and a heptadecafluorodecyl group. R⁶ is preferably a methyl group or a phenyl group.

It is preferred that 50% or more, more preferably 70% or more of all the R⁶s are methyl groups. Further, it may also be that all the R⁶s are methyl groups.

R⁷ represents an alkylene group having 1 to 5 carbon atoms, preferably an ethylene group.

q is an integer of 2 to 3. s satisfies 0≤s≤500. t and u are each a number of 0 or 1. However, in the formula (4), a sum of t and u is 1, s and u shall not both be 1 or larger at the same time.

In the formula (1), a is 1.0 to 2.5, preferably 1.2 to 2.3. When a is smaller than 1.0, there will be exhibited a poor feeling of use; when a is larger than 2.5, there will be observed a poor luster and adhesiveness.
b is 0.001 to 1.5, preferably 0.005 to 0.5. When b is smaller than 0.001, a natural makeup finish cannot be achieved, or a poor adhesiveness will be observed. When b is larger than 1.5, there will be observed a stickiness due to an excessively high hydrophilicity.
c is 0.001 to 1.5, preferably 0.05 to 1.0. When c is smaller than 0.001, an emollient effect cannot be achieved, and luster cannot be achieved either. When c is larger than 1.5, a poor feeling of use tends to be observed due to an excessively high viscosity.
d is 0 to 1.0, preferably 0 to 0.5. When d is larger than 1.0, luster and adhesiveness cannot be achieved.
a, b, c, and d satisfy 1. 1 <a+b+c+d<4, preferably 1.2<a+b+c+d<2.5.

The weight-average molecular weight of the organopolysiloxane compound represented by the formula (1) is 500 to 200,000, preferably 1,000 to 100,000. When the weight-average molecular weight is smaller than 500, a water repellency cannot be achieved, or a poor makeup durability will be observed. Further, when the weight-average molecular weight is larger than 200,000, a poor feeling of use will be exhibited, and a poor makeup finish will be observed as well. In this specification, weight-average molecular weight is a value in terms of polystyrene, that is measured in a gel permeation chromatography (GPC) analysis conducted under the following measurement conditions.

### [Measurement conditions]

Developing solvent: Tetrahydrofuran (THF)
Flow rate: 0.6 mL/min
Detector: Differential refractive index detector (RI)
Column: TSK Guardcolumn Super H-H
   TSK gel Super HM-N (6.0 mm I.D. × 15 cm × 1)
   TSK gel Super H2500 (6.0 mm I.D. × 15 cm × 1)
(All manufactured by Tosoh Corporation)
Column temperature: 40°C
Sample injection volume: 50 µL (THF solution with a concentration of 0.3% by mass)

The organopolysiloxane compound used as the component (A) in the present invention can be synthesized by reacting a primary amino group-containing organopolysiloxane with itaconic acid or an ester thereof. The primary amino group-containing organopolysiloxane may be a commercially available one, or a synthesized one. As a general synthesis method, there is a method of synthesizing a side chain alkylamino group-modified organopolysiloxane, using a siloxane oligomer and a cyclic siloxane that are obtained by hydrolyzing aminoalkylmethyldimethoxysilane, and a basic catalyst.

Further, if using bis(aminopropyl)tetramethyldisiloxane instead of the hydrolysates of aminoalkylmethyldimethoxysilane, there can be obtained a dual end aminopropyl group-modified organopolysiloxane.

The above organopolysiloxane compound can be easily and directly prepared by using itaconic acid or an ester thereof in an amount of 0.5 to 1.5 mol, preferably 1.0 mol per 1 mol of the primary amino groups, and reacting the itaconic acid or ester thereof with the primary amino functional groups at a high temperature for a sufficient amount of time. Particularly, if reacting 0.5 to 1.5 mol, preferably 0.9 to 1.1 mol of the itaconic acid or ester thereof with respect to 1 mol of the primary amino groups, all the itaconic acid will substantially react with all the primary amino functional groups whereby there will be produced an organopolysiloxane compound having at least one pyrrolidone carboxyl group and/or ester thereof.

The above reaction may be performed without a solvent or in a solvent such as an alcohol, a hydrocarbon solvent, and a chlorinated hydrocarbon solvent, preferably at a temperature of 90 to 160°C. The reaction will proceed easily, and a complete reaction between the itaconic acid or ester thereof and the primary amino groups will take place in about 1 to 5 hours. The completion of the reaction may be determined by conducting an amine value and acid value analysis, or measuring a releasing amount of water and/or alcohol as byproducts, in accordance with common methods.

By containing organic groups including a pyrrolidone carboxyl group, the organopolysiloxane compound as the component (A) is one having a high adhesiveness and emollient property to the skin. Further, by containing aromatic groups such as an aryl group or an aralkyl group, improved is a compatibility to an oily solidifying agent and an oily gelling agent that are the core elements of an oily cosmetic, whereby various oily cosmetics can be produced. In addition, since it will be an oil agent with a significantly high refractive index, a cosmetic containing the component (A) will bring about a healthy luster.

The organopolysiloxane compound as the component (A) is preferably contained in the entire cosmetic by an amount of 0.1 to 60% by mass, more preferably 1 to 40% by mass. When the component (A) is contained in an amount of smaller than 0.1% by mass, although adhesiveness may improve slightly, luster will not be sufficiently satisfactory; when the component (A) is contained in an amount of larger than 60% by mass, there will be felt a heaviness in spreadability due to an excessively high adhesiveness. Further, one kind of such organopolysiloxane compound may be used alone, or two or more kinds thereof may be used in combination, as appropriate.

### Component (B)

A component (B) used in the present invention is a hydrocarbon-based oily solidifying agent and/or a non-hydrocarbon-based oily gelling agent.

### Hydrocarbon-based oily solidifying agent

Examples of the hydrocarbon-based oily solidifying agent used as the component (B) in the present invention include a paraffin wax, a polyethylene wax, a polyethylene/polypropylene wax, a Fischer-Tropsch wax, ceresin, a microcrystalline wax, a hydrogenated polyisobutene, a candelilla wax, a carnauba wax, and a polyamide resin, of which particularly preferred are a paraffin wax, a polyethylene wax, a polyethylene/polypropylene wax, a Fischer-Tropsch wax, ceresin, and a microcrystalline wax.

### Non-hydrocarbon-based oily gelling agent

Examples of the non-hydrocarbon-based oily gelling agent used as the component (B) in the present invention include a sugar skeleton-containing fatty acid ester, a glycerin skeleton-containing fatty acid ester, a metal soap, and an organic modified clay mineral, of which preferred are a sugar skeleton-containing fatty acid ester and a glycerin skeleton-containing fatty acid ester.

The sugar skeleton-containing fatty acid ester may, for example, be a dextrin fatty acid ester and an inulin fatty acid ester, specific examples of which may include dextrin palmitate, dextrin myristate, dextrin (palmitate/ethylhexanoate), dextrin (palmitate/hexyldecanoate), sucrose stearate, and inulin stearate.

Further, the glycerin skeleton-containing fatty acid ester may, for example, be a polyglycerin fatty acid ester and a condensate of a glycerin fatty acid ester, specific examples of which may include glyceryl tri(behenate/isostearate/eicosanedioate), glyceryl (behenate/eicosanedioate), polyglyceryl-10 (behenate/eicosanedioate), and glyceryl tri(caprylate/caprate).

Examples of the metal soap include aluminum stearate and magnesium myristate. Examples of the organic modified clay mineral include dimethyldioctadecylammonium montmorillonite, dimethyldioctadecylammonium hectorite, and disteardimonium hectorite.

The component (B) is preferably contained in the entire cosmetic by an amount of 0.1 to 40% by mass. When the component (B) is contained in an amount of smaller than 0.1% by mass, there will be a problem of, for example, triggering sedimentation over time due to an insufficient hardness; when the component (B) is contained in an amount of larger than 40% by mass, an unpleasant feeling will be incurred due to an excessively hard oily cosmetic. It is more preferred that the component (B) be contained in the entire cosmetic by an amount of 1 to 20% by mass.

One kind of such hydrocarbon-based oily solidifying agent and/or non-hydrocarbon-based oily gelling agent may be used alone, or two or more kinds thereof may be used in combination.

The oily cosmetic of the present invention is suitable for use in a lip cosmetic as it is particularly superior in adhesiveness, softness, luster, and emollient property.

### Optional components

Depending on its intended use, the oily cosmetic of the present invention may contain other oil agents in addition to the above organopolysiloxane compound, oily solidifying agent, and oily gelling agent. As such oil agents, there may be employed either volatile oil agents or oil agents other than volatile oil agents (solid, semisolid or liquid oil agents) so long as they are those that are used in normal cosmetics.

The oily cosmetic of the present invention may contain a volatile silicone oil and/or a volatile organic oil, as volatile oil agents.

Examples of the volatile silicone oil include dimethylpolysiloxane (dimer, trimer, tetramer, pentamer), caprylyl methicone, octamethylcyclotetrasiloxane, decamethylcyclopentasiloxane, dodecamethylcyclohexasiloxane, tetramethyltetrahydrogencyclotetrasiloxane, tristrimethylsiloxymethylsilane, and tetrakistrimethylsiloxysilane.

The volatile organic oil may, for example, be a hydrocarbon oil and an ester oil, specific examples of which may include α-olefin oligomer, light isoparaffin, isododecane, light liquid isoparaffin, and butyl acetate.

As a volatile oil agent, preferred are dimethylpolysiloxane (tetramer, pentamer), caprylyl methicone, tristrimethylsiloxymethylsilane, tetrakistrimethylsiloxysilane, light isoparaffin, isododecane, light liquid isoparaffin, and butyl acetate.

These volatile oil agents are preferably contained in the entire cosmetic by an amount of 1 to 98% by mass, more preferably 1 to 50% by mass, even more preferably 3 to 30% by mass.

In addition to the above volatile oil agents, the oily cosmetic of the present invention may further contain other oil agents that are permissible in cosmetic products. As such oil agents other than volatile oil agents, there may be listed, for example, a polar oil such as a non-volatile silicone oil, a higher fatty acid, a higher alcohol, an ester oil, a glyceride oil, and a natural animal and vegetable oil; a semisynthetic oil; and/or a fluorine-based oil. One kind of these oil agents may be used alone, or two or more kinds of them may be used in combination.

Examples of a silicone oil include linear or branched organopolysiloxanes such as non-volatile dimethylpolysiloxane, phenyl trimethicone, methylphenylpolysiloxane, dimethylsiloxyphenyl trimethicone, diphenylsiloxyphenyl trimethicone (e.g., KF-56A by Shin-Etsu Chemical Co., Ltd.), methylhexylpolysiloxane, methylhydrogenpolysiloxane, and a dimethylsiloxane-methylphenylsiloxane copolymer; tetramethyltetraphenylcyclotetrasiloxane; an amino-modified organopolysiloxane; cyclic siloxane solutions of silicone rubbers, silicones, and rubbers, such as a dimethylpolysiloxane with a high polymerization degree, a gum-like amino-modified organopolysiloxane, and a gum-like dimethylsiloxane-methylphenylsiloxane copolymer; trimethylsiloxy silicate; a cyclic organopolysiloxane of trimethylsiloxy silicate; higher alkoxy-modified organopolysiloxanes such as stearoxysilicone; higher fatty acid-modified organopolysiloxanes; alkyl-modified organopolysiloxanes; long-chain alkyl-modified organopolysiloxanes; fluorine-modified organopolysiloxanes; silicone resins; and silicone resin materials.

Examples of a higher fatty acid include lauric acid, myristic acid, palmitic acid, stearic acid, behenic acid, undecylenic acid, oleic acid, linoleic acid, linolenic acid, arachidonic acid, eicosapentaenoic acid (EPA), docosahexaenoic acid (DHA), isostearic acid, and 12-hydroxystearic acid.

Examples of a higher alcohol include lauryl alcohol, myristyl alcohol, palmityl alcohol, stearyl alcohol, behenyl alcohol, hexadecyl alcohol, oleyl alcohol, isostearyl alcohol, hexyldodecanol, octyldodecanol, cetostearyl alcohol, 2-decyltetradecinol, cholesterol, phytosterol, POE cholesterol ether, monostearyl glycerin ether (batyl alcohol), and monooleyl glyceryl ether (serachyl alcohol).

Examples of an ester oil include diisobutyl adipate, 2-hexyldecyl adipate, di-2-heptylundecyl adipate, N-alkyl glycol monoisostearate, isocetyl isostearate, trimethylolpropane triisostearate, ethylene glycol di-2-ethylhexanoate, cetyl 2-ethylhexanoate, trimethylolpropane tri-2-ethylhexanoate, pentaerythritol tetra-2-ethylhexanoate, cetyl octanoate, octyldodecyl gum ester, oleyl oleate, octyldodecyl oleate, decyl oleate, neopentyl glycol dioctoate, neopentyl glycol dicaprate, propylene glycol dicaprate, coconut alkyl (caprate/caprylate), triethyl citrate, 2-ethylhexyl succinate, isocetyl stearate, butyl stearate, diisopropyl sebacate, di-2-ethylhexyl sebacate, ethylhexyl methoxycinnamate, cetyl lactate, myristyl lactate, isononyl isononanoate, isotridecyl isononanoate, isopropyl palmitate, 2-ethylhexyl palmitate, 2-hexyldecyl palmitate, 2-heptylundecyl palmitate, cholesteryl 12-hydroxystearate, dipentaerythritol fatty acid ester, isopropyl myristate, octyldodecyl myristate, 2-hexyldecyl myristate, myristyl myristate, hexyldecyl dimethyl octanoate, ethyl laurate, hexyl laurate, N-lauroyl-L-glutamic acid-2-octyldodecyl ester, lauroyl sarcosine isopropyl ester, and diisostearyl malate.

Examples of a glyceride oil include acetoglyceryl, glyceryl triisooctanoate, glyceryl triisostearate, glyceryl triisopalmitate, glyceryl monostearate, glyceryl di-2-heptylundecanoate, glyceryl trimyristate, diglyceryl isostearate myristate, and glyceryl triethylhexanoate.

Further, examples of a natural animal and vegetable oil and a semisynthetic oil include natural vegetable oils such as avocado oil, linseed oil, almond oil, privet wax, perilla oil, olive oil, cacao butter, kapok wax, kaya oil, carnauba wax, candelilla wax, apricot kernel oil, wheat germ oil, sesame oil, rice germ oil, rice bran oil, sugar cane wax, sasanqua oil, safflower oil, shea butter, Chinese paulownia oil, cinnamon oil, jojoba wax, squalane, squalene, shellac wax, soybean oil, tea seed oil, camellia oil, evening primrose oil, corn oil, rape seed oil, Japanese paulownia oil, bran wax, germ oil, persic oil, palm oil, palm kernel oil, castor oil, sunflower oil, grape seed oil, bayberry wax, jojoba oil, macadamia nut oil, beeswax, meadowfoam oil, cottonseed oil, cotton wax, Japan wax, Japan wax kernel oil, montan wax, coconut oil, and peanut oil; natural animal oils such as liver oil, beef fat, beef foot fat, beef bone fat, hardened beef fat, whale wax, hardened oil, turtle oil, pork fat, horse fat, mink oil, refined candelilla wax, sheep fat, lanolin, liquid lanolin, and egg yolk oil; hardened castor oil; castor oil fatty acid methyl ester; hydrogenated jojoba oil; hardened coconut oil; tri-coconut oil fatty acid glyceride; reduced lanolin; lanolin alcohol; hard lanolin; lanolin acetate; lanolin alcohol acetate; and lanolin fatty acid isopropyl.

Examples of a fluorine-based oil agent include perfluoropolyether, perfluorodecalin, and perfluorooctane.

Although depending on the agent system of the cosmetic, the added amount of the oil agents other than volatile oil agents that are permissible in the oily cosmetic of the present invention is preferably 1 to 98% by mass, more preferably 1 to 50% by mass, even more preferably 3 to 30% by mass, with respect to the entire cosmetic.

As ingredients used in normal cosmetics, the oily cosmetic of the present invention may contain, for example, a powder, a membrane-forming agent, a surfactant, a powder, a resin, an ultraviolet absorber, a preservative, an antioxidant, and a fragrance. Further, to the extent that the effects of the present invention are impaired, the oily cosmetic of the present invention may contain, for example, water, alcohols, water-soluble polymers, a moisturizer, an antibacterial agent, salts, a pH adjustor, a chelating agent, a refreshing agent, an antiinflammatory agent, a skin-beautifying ingredient (e.g., whitening agent, cell activating agent, skin problem improving agent, blood circulation promoting agent, skin astringent agent, and anti-seborrheic agent), vitamins, amino acids, nucleic acids, hormones, and an inclusion compound.

Further, the oily cosmetic of the present invention may be one containing the above cosmetic ingredients, such as a makeup cosmetic including liquid foundation, oil-based foundation, cheek rouge, eye shadow, mascara, eyeliner, eyebrow cosmetic, and lipstick; and an ultraviolet-protective cosmetic including sunscreen oil, sunscreen emulsion, and sunscreen cream.

### EXAMPLES

The present invention is described in greater detail hereunder with reference to production, comparative production, working, and comparative examples; however, the present invention shall not be limited to these working examples. Here, in the following formulae, C₆H₅- represents a phenyl group. Further, "%" refers to "% by mass."

### [Production example 1]

Here, 740 g of an amino group-containing organopolysiloxane represented by the following composition formula was added to a separable flask.

[Chemical formula 10] (CH₃)_{1.54}(NH₂CH₂CH₂CH₂)_{0.036}(C₆H₅)_{0.5}SiO_{0.962}

Next, 34 g of itaconic acid was added thereto, where stirring was performed at 140°C for 3 hours, and an ester adapter was used to distill away a theoretical amount of water. After removing unreacted products via stripping under a reduced pressure, there was obtained an organopolysiloxane compound having the following structure at a yield of 95%. The light yellow transparent liquid obtained had a weight-average molecular weight of 3,000.

[Chemical formula 11] (CH₃)_{1.54}(R^{Z})_{0.036}(C₆H₅)_{0.5}SiO_{0.962}

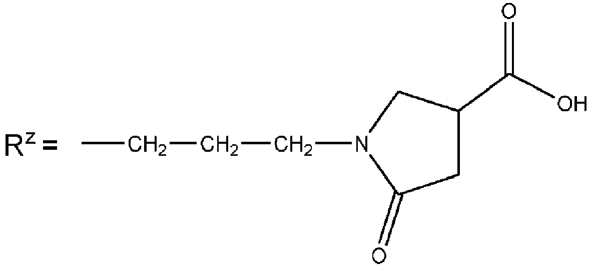

### [Production example 2]

Here, 200 g of an amino group-containing organopolysiloxane represented by the following composition formula was added to a separable flask.

[Chemical formula 12] (CH₃)_{1.61}(NH₂CH₂CH₂CH₂)_{0.009}(C₆H₅)_{0.39}SiO_{0.996}

Next, 5.0 g of itaconic acid was added thereto, where stirring was performed at 140°C for 3 hours, and an ester adapter was used to distill away a theoretical amount of water. After removing unreacted products via stripping under a reduced pressure, there was obtained an organopolysiloxane compound having the following structure at a yield of 96%. The light yellow transparent liquid obtained had a weight-average molecular weight of 11,000.

[Chemical formula 13] (CH₃)_{1.61}(R^{Z})_{0.009}(C₆H₅)_{0.39}SiO_{0.996}

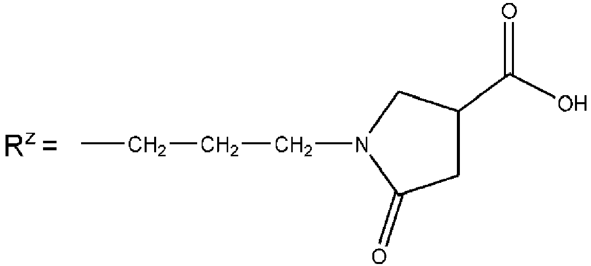

### [Production example 3]

Here, 500 g of an amino group-containing organopolysiloxane represented by the following composition formula was added to a separable flask.

[Chemical formula 14] (CH₃)_{1.82}(NH₂CH₂CH₂CH₂)_{0.013}(CₑH₅)_{0.18}Si0_{0.994}

Next, 12.5 g of dimethyl itaconate was added thereto, where stirring was performed at 100°C for 3 hours, and an ester adapter was used to distill away a theoretical amount of methanol. After removing unreacted products via stripping under a reduced pressure, there was obtained an organopolysiloxane compound having the following structure at a yield of 95%. The light yellow transparent liquid obtained had a weight-average molecular weight of 20,000.

[Chemical formula 15] (CH₃)_{1.82}(R^{Z1})_{0.013}(C₆H₅)_{0.18}SiO_{0.994}

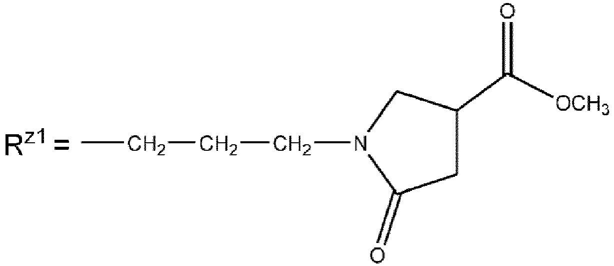

### [Production example 4]

Here, 300 g of an amino group-containing organopolysiloxane represented by the following composition formula was added to a separable flask.

[Chemical formula 16] (CH₃)_{1.61}(n-C₁₂H₂₅)_{0.076}(NH₂CH₂CH₂CH₂)_{0.091}(C₆H₅)_{0.30}(R^{Z2})_{0.076}SiO_{0.924}

Next, 34.5 g of itaconic acid was added thereto, where stirring was performed at 140°C for 3 hours, and an ester adapter was used to distill away a theoretical amount of water. After removing unreacted products via stripping under a reduced pressure, there was obtained an organopolysiloxane compound having the following structure at a yield of 95%. The light yellow transparent liquid obtained had a weight-average molecular weight of 1,300.

[Chemical formula 17] (CH₃)_{1.61}(n-C₁₂H₂₅)_{0.076}(R^{Z})_{0.091}(C₆H₅)_{0.30}(R^{Z2})_{0.076}SiO_{0.924}

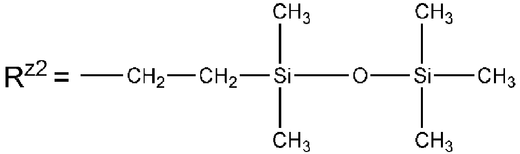

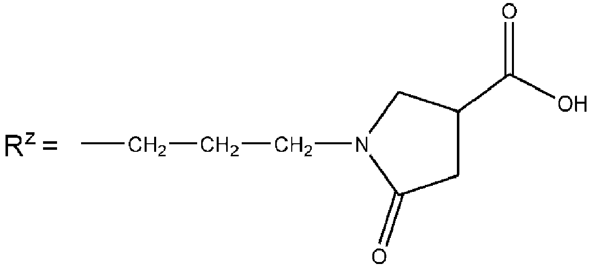

### [Comparative production example 1]

Here, 500 g of an amino group-containing organopolysiloxane represented by the following composition formula was added to a separable flask.

[Chemical formula 18] (CH₃)_{2.01}(NH₂CH₂CH₂CH₂)_{0.016}SiO_{0.987}

Next, 15 g of itaconic acid was added thereto, where stirring was performed at 140°C for 3 hours, and an ester adapter was used to distill away a theoretical amount of water. After removing unreacted products via stripping under a reduced pressure, there was obtained an organopolysiloxane compound having the following structure at a yield of 95%. The light yellow transparent liquid obtained had a weight-average molecular weight of 5,500.

[Chemical formula 19] (CH₃)_{2.01}(R^{Z})_{0.016}SiO_{0.987}

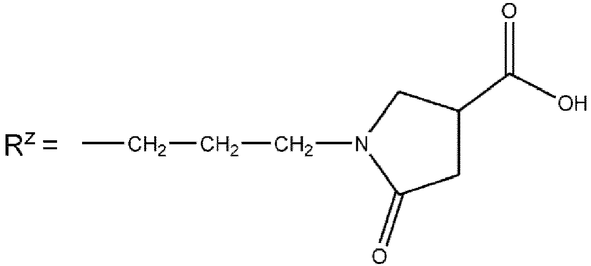

### Working examples 1 to 4 and comparative examples 1 to 3; stick-shaped lipsticks

A stick-shaped lipstick was produced at the compositions shown in Table 1 and in accordance with the following production method. The formability, feeling of use, and usability thereof were evaluated by the following evaluation method. The results are shown in Table 1 as well.

### (Production method)

A: Ingredients 1 to 10 are uniformly melted by being heated to 90°C.
B: Ingredients 11 to 16 are uniformly mixed and subjected to a roll treatment.
C: The product obtained in the step B and an ingredient 17 are added to and uniformly mixed with the product obtained in the step A.
D: After defoaming the product obtained in the step C, the product is poured into a container and then cooled for solidification to obtain a stick-shaped lipstick.

### (Evaluation method)

As for the product obtained as above, the formability thereof as a stick-shaped lipstick was confirmed, followed by using such formed lipstick to conduct a use test with a specialized panel of 50 female participants, where the product was comprehensively evaluated by judging on average points of evaluations performed under the criteria below with regard to spreadability, smoothness, adhesiveness, and fitting property at the time of application; non-stickiness, moistness, and finishing luster after finishing the makeup; color transfer-freeness; color fade-freeness; smudge-freeness; and makeup durability.

### [Confirmation of formability]

⊚: Successfully formed
×: Forming failed

### [Evaluation criteria]

5 points: Very favorable
4 points: favorable
3 points: Normal
2 points: Slightly unfavorable
1 point: Unfavorable

### [Judgment]

⊚: Average score 4.5 or higher
∘: Average score 3.5 or higher, but lower than 4.5
Δ: Average score 2.5 or higher, but lower than 3.5
×: Average score lower than 2.5

**[Table 1]**

| | Ingredient | Working example 1 | Working example 2 | Working example 3 | Working example 4 | Comparative example 1 | Comparative example 2 | Comparative example 3 |
|---|---|---|---|---|---|---|---|---|
| 1 | Fischer-Tropsch wax | 8 | 8 | 8 | 8 | 8 | 8 | 8 |
| 2 | Ceresin | 4 | 4 | 4 | 4 | 4 | 4 | 4 |
| 3 | Hydrogenated polyisobutene | 30 | 30 | 30 | 30 | 30 | 30 | 30 |
| 4 | Diisostearyl malate | 10 | 10 | 10 | 10 | 10 | 10 | 10 |
| 5 | Neopentyl glycol dicaprate | 8 | 8 | 8 | 8 | 8 | 8 | 38 |
| 6 | Organopolysiloxane obtained in production example 1 | 30 | - | - | 15 | - | - | - |
| 7 | Organopolysiloxane obtained in production example 2 | - | 30 | - | - | - | - | - |
| 8 | Organopolysiloxane obtained in production example 3 | - | - | 30 | 15 | - | - | - |
| 9 | Organopolysiloxane obtained in comparative production example 1 | - | - | - | - | 30 | - | - |
| 10 | Methylphenylpolysiloxane * | - | - | - | - | - | 30 | - |
| 11 | Red No.201 | 1 | 1 | 1 | 1 | 1 | 1 | 1 |
| 12 | Red No.202 | 2 | 2 | 2 | 2 | 2 | 2 | 2 |
| 13 | Yellow No.4 aluminum lake | 1 | 1 | 1 | 1 | 1 | 1 | 1 |
| 14 | Titanium oxide | 1 | 1 | 1 | 1 | 1 | 1 | 1 |
| 15 | Pearly pigment | 5 | 5 | 5 | 5 | 5 | 5 | 5 |
| 16 | Fragrance | Moderate amount | Moderate amount | Moderate amount | Moderate amount | Moderate amount | Moderate amount | Moderate amount |
| | | | | | | | | |

| Evaluation item | | | | | | | | |
|---|---|---|---|---|---|---|---|---|
| 1 | Formability | ⊚ | ⊚ | ⊚ | ⊚ | × | ⊚ | ⊚ |
| 2 | Spreadability at the time of application | ⊚ | ○ | ⊚ | ⊚ | - | ⊚ | ⊚ |
| 3 | Smoothness at the time of application | ⊚ | ⊚ | ⊚ | ⊚ | - | Δ | ⊚ |
| 4 | Adhesiveness | ⊚ | ⊚ | ⊚ | ⊚ | - | Δ | Δ |
| 5 | Fitting property | ⊚ | ⊚ | ○ | ⊚ | - | Δ | × |
| 6 | Non-stickiness | ⊚ | ⊚ | ⊚ | ⊚ | - | ⊚ | Δ |
| 7 | Moistness | ⊚ | ⊚ | ⊚ | ⊚ | - | Δ | Δ |
| 8 | Finishing luster | ⊚ | ⊚ | ⊚ | ⊚ | - | ⊚ | × |
| 9 | Color transfer-freeness | ⊚ | ⊚ | ⊚ | ⊚ | - | Δ | × |
| 10 | Color fade-freeness | ⊚ | ⊚ | ⊚ | ⊚ | - | Δ | × |
| 11 | Smudge-freeness | ⊚ | ⊚ | ⊚ | ⊚ | - | Δ | × |
| 12 | Makeup durability | ⊚ | ⊚ | ⊚ | ⊚ | - | Δ | × |
| 13 | Comprehensive evaluation | ⊚ | ⊚ | ⊚ | ⊚ | × | Δ | Δ |

| | | | | | | | | |
|---|---|---|---|---|---|---|---|---|
| (*)KF-54 by Shin-Etsu Chemical Co., LTD. | | | | | | | | |

As are clear from the results shown in Table 1, and as compared to the comparative example 2, the stick-shaped lipsticks of the working examples 1 to 4 each containing the siloxane compound of the present invention exhibited an excellent smoothness, adhesiveness, and fit at the time of application, were moist, and also exhibited a significantly excellent makeup durability without showing color transfer, color fade, and smudge. Further, even as compared to the comparative example 3, the lipsticks of the working examples exhibited an excellent adhesiveness and fit at the time of application, a significant luster due to a moistness, and a significantly excellent makeup durability without showing color transfer, color fade, and smudge. Here, in the case of the comparative example 1 where there was used a phenyl group-free pyrrolidone carboxyl group-containing polysiloxane, the product failed to be formed into the shape of a stick.

Moreover, an emollient effect was observed with the stick-shaped lipsticks of the working examples 1 to 4; however, no emollient effect was observed with the comparative examples 1 to 3.

### Working example 5: lipstick

**[Table 2]**

| (Ingredient) | | (%) |
|---|---|---|
| | 1. Inulin stearate | 6.0 |
| | 2. Dextrin palmitate | 3.0 |
| | 3. Polyethylene wax | 1.5 |
| | 4. Polyglyceryl-2 triisostearate | 15.0 |
| | 5. Diisostearyl malate | 10.0 |
| | 6. Organopolysiloxane obtained in production example 1 | 25.0 |
| | 7. Propylene glycol dicaprate | 20.0 |
| | 8. Dissolved product of trimethylsiloxy silicate (Note 3) | 5.0 |
| | 9. Isotridecyl isononanoate | Remaining amount |
| | 10. Silicone-branched polyglycerin-modified silicone (Note 4) | 0.5 |
| | 11. Red No.201 | Moderate amount |
| | 12. Yellow No.4 aluminum lake | Moderate amount |
| | 13. Silicone-treated titanium oxide (Note 5) | Moderate amount |
| | 14. Silicone-treated colcothar (Note 5) | Moderate amount |
| | 15. Silicone-treated yellow iron oxide (Note 5) | Moderate amount |
| | 16. Silicone-treated black iron oxide (Note 5) | Moderate amount |
| | 17. Fragrance | Moderate amount |
| | Total | 100.0 |

| | | |
|---|---|---|
| (Note 3) KF-7312J by Shin-Etsu Chemical Co., Ltd. (Dissolved product containing decamethylcyclopentasiloxane 50% + silicone resin 50%) (Note 4) KF-6106 by Shin-Etsu Chemical Co., Ltd. (Note 5) KTP-09W, R, Y, B by Shin-Etsu Chemical Co., Ltd. (KF-9909 treatment coloring inorganic pigment, W: White, R: Red, Y: Yellow, B: Black) | | |

### (Production method in working example 5)

A: Ingredients 1 to 8 are melted by heating.
B: Ingredients 9 to 16 are uniformly mixed and subjected to a roll treatment.
C: The product obtained in the step B is added to and uniformly mixed with the product obtained in the step A.
D: After defoaming the product obtained in the step C, ingredient 17 is added thereto before loading the product into a container to obtain a lipstick.

The lipstick (working example 5) of the present invention obtained as above was one exhibiting superior features including: an excellent smoothness at the time of application; a light spreadability without stickiness; an excellent adhesiveness; a lustrous beautiful makeup finish with a favorable fit; a favorable makeup durability without showing smudge, color transfer, and color fade; and achieving a non-dry and non-chapped lip.

### Working example 6: lipstick

**[Table 3]**

| (Ingredient) | | (%) |
|---|---|---|
| | 1. Polyethylene wax | 11.0 |
| | 2. Ceresin | 4.0 |
| | 3. Diisostearyl malate | 25.0 |
| | 4. Organopolysiloxane obtained in production example 4 | 15.0 |
| | 5. Macadamia nut oil polyglyceryl-6 ester behenate | 10.0 |
| | 6. Polyglyceryl-10 pentaisostearate | 12.0 |
| | 7. Glyceryl tri(caprylate/caprate) | Remaining amount |
| | 8. Isotridecyl isononanoate | 3.0 |
| | 9. Silicone-modified acrylic polymer (Note 2) | 0.5 |
| | 10. Red No.226 | Moderate amount |
| | 11. Yellow No.4 aluminum lake | Moderate amount |
| | 12. Acrylic silicone-treated titanium oxide (Note 6) | Moderate amount |
| | 13. Acrylic silicone-treated colcothar (Note 6) | Moderate amount |
| | 14. Acrylic silicone-treated yellow iron oxide (Note 6) | Moderate amount |
| | 15. Acrylic silicone-treated black iron oxide (Note 6) | Moderate amount |
| | 16. Pearly pigment | Moderate amount |
| | Total | 100.0 |

| | | |
|---|---|---|
| (Note 2) KP-578 by Shin-Etsu Chemical Co., Ltd. (Note 6) treated with KP-574 by Shin-Etsu Chemical Co., Ltd. | | |

### (Production method in working example 6)

A: Ingredients 1 to 7 are melted by heating.
B: Ingredients 8 to 15 are uniformly mixed and subjected to a roll treatment.
C: The product obtained in the step B and ingredient 16 are added to and uniformly mixed with the product obtained in the step A.
D: After defoaming the product obtained in the step C, the product is loaded into a container to obtain a lipstick.

The lipstick (working example 6) of the present invention obtained as above was one exhibiting superior features including: an excellent smoothness at the time of application; a light spreadability without stickiness; an excellent adhesiveness; a lustrous beautiful makeup finish with a favorable fit; a favorable makeup durability without showing smudge, color transfer, and color fade; and achieving a non-dry and non-chapped lip.

### Working example 7: lip gloss

**[Table 4]**

| (Ingredient) | | (%) |
|---|---|---|
| | 1. Glyceryl tri(behenate/isostearate/eicosanedioate) | 8.0 |
| | 2. Organopolysiloxane obtained in production example 4 | 50.0 |
| | 3. Hydrogenated polyisobutene | 25.0 |
| | 4. Dissolved product of acrylic silicone (Note 7) | 3.0 |
| | 5. Isotridecyl isononanoate | Remaining amount |
| | 6. Silicone-modified acrylic polymer (Note 2) | 0.3 |
| | 7. Red No.202 | Moderate amount |
| | 8. Yellow No.4 aluminum lake | Moderate amount |
| | 9. Silicone-treated titanium oxide (Note 8) | Moderate amount |
| | 10. Silicone-treated black iron oxide (Note 8) | Moderate amount |
| | 11. Pearly pigment | Moderate amount |
| | Total | 100.0 |

| | | |
|---|---|---|
| (Note 7) KP-545 by Shin-Etsu Chemical Co., Ltd. (Dissolved product containing decamethylcyclopentasiloxane 70% + acrylic silicone 30%) (Note 8) treated with KF-99P by Shin-Etsu Chemical Co., Ltd. | | |

### (Production method in working example 7)

A: Ingredients 1 to 5 are melted by heating.
B: Ingredients 6 to 10 are uniformly mixed and subjected to a roll treatment.
C: The product obtained in the step B and ingredient 11 are added to and uniformly mixed with the product obtained in the step A.
D: After defoaming the product obtained in the step C, the product is loaded into a container to obtain a lip gloss.

The lip gloss (working example 7) of the present invention obtained as above was one exhibiting superior features including: an excellent spreadability and smoothness at the time of application; non-stickiness; an excellent adhesiveness; a lustrous beautiful makeup finish with a favorable fit; a favorable makeup durability without showing smudge, color transfer, and color fade; and achieving a non-dry and non-chapped lip.

### Working example 8: oily solid foundation

**[Table 5]**

| (Ingredient) | | (%) |
|---|---|---|
| | 1. Polyethylene wax | 1.0 |
| | 2. Microcrystalline wax | 3.0 |
| | 3. Candelilla wax | 0.5 |
| | 4. Organopolysiloxane obtained in production example 1 | 10.0 |
| | 5. Dimethylsiloxyphenyl trimethicone (Note 9) | 3.0 |
| | 6. Ethylhexyl methoxycinnamate | 8.0 |
| | 7. Dimethylpolysiloxane (2cs) | 5.0 |
| | 8. Isotridecyl isononanoate | Remaining amount |
| | 9. Spherical silicone composite powder (Note 10) | 3.0 |
| | 10. Polymethylsilsesquioxane (Note 11) | 7.0 |
| | 11. 2-Ethylhexyl palmitate | 6.0 |
| | 12. Silicone-branched polyglycerin-modified silicone (Note 4) | 1.0 |
| | 13. Silicone-treated fine particle titanium oxide (Note 12) | 7.0 |
| | 14. Silicone-treated titanium oxide (Note 13) | Moderate amount |
| | 15. Silicone-treated colcothar (Note 13) | Moderate amount |
| | 16. Silicone-treated yellow iron oxide (Note 13) | Moderate amount |
| | 17. Silicone-treated black iron oxide (Note 13) | Moderate amount |
| | Total | 100.0 |

| | | |
|---|---|---|
| (Note 9) KF-56A by Shin-Etsu Chemical Co., Ltd. (Note 10) KSP-100 by Shin-Etsu Chemical Co., Ltd. (silicone resin-coated silicone rubber composite powder with average particle size of 5 µm) (Note 11) KMP-591 by Shin-Etsu Chemical Co., Ltd. (Note 12) treated with KF-9901 by Shin-Etsu Chemical Co., Ltd. (Note 13) treated with AES-3083 by Shin-Etsu Chemical Co., Ltd. | | |

### (Production method in working example 8)

A: Ingredients 1 to 8 are melted by heating.
B: Ingredients 11 to 17 are uniformly mixed and subjected to a roll treatment.
C: The product obtained in the step B and ingredients 9 and 10 are added to and uniformly mixed with the product obtained in the step A.
D: After defoaming the product obtained in the step C, the product is loaded into a container to obtain a solid foundation.

The oily solid foundation (working example 8) of the present invention obtained as above was a significantly superior one in that it exhibited features including: a light spreadability; a favorable fit to the skin; a moist makeup finish; achieving a lustrous and resilient makeup membrane; and a favorable makeup durability.

### Working example 9: oily foundation

**[Table 6]**

| (Ingredient) | | (%) |
|---|---|---|
| | 1. Cross-linked dimethylpolysiloxane mixture (Note 14) | 5.0 |
| | 2. Organopolysiloxane obtained in production example 4 | 5.0 |
| | 3. Silicone-branched alkyl polyether co-modified silicone (Note 15) | 3.0 |
| | 4. Disteardimonium hectorite | 2.0 |
| | 5. Silylated silica | 1.0 |
| | 6. Dissolved product of silicone-modified pullulan (Note 16) | 2.0 |
| | 7. Dimethylpolysiloxane (6cs) | 5.0 |
| | 8. Ethanol | 8.0 |
| | 9. Isododecane | Remaining amount |
| | 10. Isotridecyl isononanoate | 10.0 |
| | 11. Silicone-modified acrylic polymer (Note 2) | 1.0 |
| | 12. Silicone-treated fine particle titanium oxide (Note 12) | 8.0 |
| | 13. Silicone-treated fine particle zinc oxide (Note 12) | 5.0 |
| | 14. Silicone-treated titanium oxide (Note 5) | Moderate amount |
| | 15. Silicone-treated colcothar (Note 5) | Moderate amount |
| | 16. Silicone-treated yellow iron oxide (Note 5) | Moderate amount |
| | 17. Silicone-treated black iron oxide (Note 5) | Moderate amount |
| | Total | 100.0 |

| | | |
|---|---|---|
| (Note 14) KSG-16 by Shin-Etsu Chemical Co., Ltd. (mixture of dimethylpolysiloxane 70 to 80% + cross-linked dimethylpolysiloxane 20 to 30%) (Note 15) KF-6038 by Shin-Etsu Chemical Co., Ltd. (Note 16) TSPL-30-ID by Shin-Etsu Chemical Co., Ltd. (mixture of isododecane 70% + silicone-modified pullulan 30%) | | |

### (Production method in working example 9)

A: Ingredients 1 to 9 are uniformly mixed.
B: Ingredients 10 to 17 are uniformly mixed and subjected to a roll treatment.
C: The product obtained in the step B is added to and uniformly mixed with the product obtained in the step A.
D: After defoaming the product obtained in the step C, the product is loaded into a container to obtain an oily foundation.

The oily foundation (working example 9) of the present invention obtained as above was a significantly superior one in that it exhibited features including: a light spreadability; a favorable fit to the skin; a moist makeup finish; achieving a lustrous and resilient makeup membrane; and a favorable makeup durability.

### Working example 10: oily mascara

**[Table 7]**

| (Ingredient) | | (%) |
|---|---|---|
| | 1. Paraffin wax | 20.0 |
| | 2. Microcrystalline wax | 8.0 |
| | 3. Polyethylene wax | 3.0 |
| | 4. Inulin stearate | 1.0 |
| | 5. Organopolysiloxane obtained in production example 1 | 8.0 |
| | 6. Disteardimonium hectorite | 2.0 |
| | 7. Silicone-branched polyether-modified silicone (Note 17) | 1.0 |
| | 8. Dissolved product of silicone-modified polynorbornene (Note 18) | 3.0 |
| | 9. Hydrogenated polyisobutene | Remaining amount |
| | 10. Neopentyl glycol dicaprate | 5.0 |
| | 11. Silicone-branched polyglycerin-modified silicone (Note 3) | 1.0 |
| | 12. Silicone-treated titanium oxide (Note 5) | Moderate amount |
| | 13. Silicone-treated colcothar (Note 5) | Moderate amount |
| | 14. Silicone-treated yellow iron oxide (Note 5) | Moderate amount |
| | 15. Silicone-treated black iron oxide (Note 5) | Moderate amount |
| | Total | 100.0 |

| | | |
|---|---|---|
| (Note 17) KF-6028 by Shin-Etsu Chemical Co., Ltd. (Note 18) NBN-30-ID by Shin-Etsu Chemical Co., Ltd. (mixture of isododecane 70% + silicone-modified polynorbornene 30%) | | |

### (Production method in working example 10)

A: Ingredients 1 to 9 are melted by heating.
B: Ingredients 10 to 15 are uniformly mixed and subjected to a roll treatment.
C: The product obtained in the step B is added to and uniformly mixed with the product obtained in the step A.
D: After defoaming the product obtained in the step C, the product is loaded into a container to obtain an oily mascara.

The oily mascara (working example 10) of the present invention obtained as above was confirmed to be a significantly superior one in that it exhibited features including: an excellent smoothness at the time of application; a light spreadability without stickiness; an excellent adhesiveness; a lustrous beautiful makeup finish with a favorable fit; and a favorable makeup durability without showing smudge.

## Claims

1. An oily cosmetic comprising the following components (A) and (B):
(A) an organopolysiloxane compound that is represented by the following general formula (1) and has a weight-average molecular weight of 500 to 200,000,
[Chemical formula 1]
R¹ₐR²_{b}R³_{c}R⁴_{d}SiO_{(4-a-b-c-d)/2}··· (1)
wherein R¹ independently represents an alkyl group having 1 to 16 carbon atoms, or a fluorine-substituted alkyl group having 3 to 12 carbon atoms; R² independently represents an organic group expressed by the following general formula (2); R³ independently represents an aryl group having 6 to 12 carbon atoms, or an aralkyl group having 7 to 14 carbon atoms; R⁴ independently represents an organopolysiloxane group expressed by the following general formula (3) or (4); a, b, c and d satisfy 1.0≤a≤2.5, 0.001≤b≤1.5, 0.001≤c≤1.5, 0≤d≤1.0, and 1.1<a+b+c+d<4, 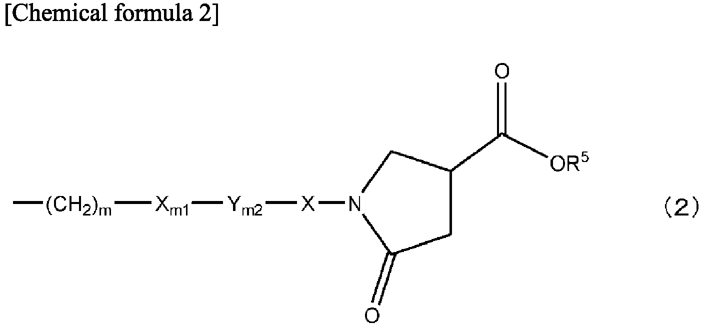 wherein R⁵ represents a hydrogen atom, a lower alkyl group having 1 to 6 carbon atoms, or an alkali metal cation; X independently represents a linear or branched alkylene group having 1 to 12 carbon atoms; Y represents -NR'-, a sulfur atom, or an oxygen atom, in which R' represents a hydrogen atom or a lower alkyl group having 1 to 6 carbon atoms; m is 0 or 2, m1 is 0 or 1, and m2 is 0 or 1, provided that when m is 0 and m2 is 1, m1 is 1, that when m is 2 and m2 is 1, m1 is 0 or 1, and that when m is 2 and m2 is 0, m1 is 0,
[Chemical formula 3]
MM^{R}Dₛ (3)
M_{q}DₛD^{R}ₜT^{R}ᵤ (4)
wherein M=R⁶₃SiO_{1/2}, M^{R}=R⁶₂R⁷SiO_{1/2}, D=R⁶₂SiO_{2/2}, D^{R}=R⁶R⁷SiO_{2/2}, T^{R}=R⁷SiO_{3/2}; R⁶ independently represents a group selected from an alkyl group having 1 to 6 carbon atoms, an aryl group having 6 to 12 carbon atoms, an aralkyl group having 7 to 14 carbon atoms, and a fluorine-substituted alkyl group having 3 to 12 carbon atoms; R⁷ independently represents an alkylene group having 1 to 5 carbon atoms; q is an integer of 2 to 3; s satisfies 0≤s≤500; t and u are each a number of 0 or 1, provided that in the formula (4), a sum of t and u is 1, and s and u shall not both be 1 or larger at the same time;
(B) a hydrocarbon-based oily solidifying agent and/or a non-hydrocarbon-based oily gelling agent.

2. The oily cosmetic according to claim 1, wherein the hydrocarbon-based oily solidifying agent as the component (B) is at least one kind of oily solidifying agent selected from a paraffin wax, a polyethylene wax, a polyethylene/polypropylene wax, a Fischer-Tropsch wax, ceresin, and a microcrystalline wax.

3. The oily cosmetic according to claim 1, wherein the non-hydrocarbon-based oily gelling agent as the component (B) is at least one kind of oily gelling agent selected from a sugar skeleton-containing fatty acid ester and a glycerin skeleton-containing fatty acid ester.

4. The oily cosmetic according to any one of claims 1 to 3, wherein the oily cosmetic is a lip cosmetic.
